**Europäisches Patentamt**

**European Patent Office**

**Office européen des brevets**

(19)

(11) Numéro de publication: **0 253 718
B1**

(12) **FASCICULE DE BREVET EUROPEEN**

(45) Date de publication du fascicule du brevet:
**07.11.90**

(51) Int. Cl.⁵: **A47G 25/90**, A61B 19/04,
G21F 7/04

(21) Numéro de dépôt: **87401594.4**

(22) Date de dépôt: **07.07.87**

(54) **Machine à enlever des protections telles que des gants ou surbottes en milieu hostile.**

(30) Priorité: **09.07.86 FR 8610002**

(43) Date de publication de la demande:
**20.01.88 Bulletin 88/3**

(45) Mention de la délivrance du brevet:
**07.11.90 Bulletin 90/45**

(84) Etats contractants désignés:
**BE DE GB IT**

(56) Documents cités:
**US-A- 1 938 685
US-A- 1 996 377
US-A- 3 695 493**

(73) Titulaire: **COMMISSARIAT A L'ENERGIE ATOMIQUE,
31/33, rue de la Fédération, F-75015 Paris(FR)**

(72) Inventeur: **Claraz, Paul, 20, rue Docteur Jeune,
F-26700 Montémilar(FR)**
Inventeur: **Caizergues, Bernard, La Louriette Quartier de
la Mirandole, F-30130 Pont Saint Esprit(FR)**
Inventeur: **Letizi, Michel, 13, Cours de la Libération,
F-84830 Sévignan du Comtat(FR)**
Inventeur: **Counit, Georges, 87, Impasse la Lavande des
Montjards, F-26700 Pierrelatte(FR)**

(74) Mandataire: **Mongrédien, André et al, c/o
BREVATOME 25, rue de Ponthieu, F-75008 Paris(FR)**

## Description

La présente invention concerne une machine à enlever des protections, telles que des gants ou des surbottes et plus particulièrement une machine à enlever des protections comprenant au moins une boîte d'enlèvement, une paroi comportant des perforations, située dans ladite boîte d'enlèvement et délimitant dans celle-ci d'une part un volume de réception comportant une zone d'entrée donnant sur l'extérieur de la boîte d'enlèvement pour recevoir un membre humain revêtu d'une protection et, d'autre part un volume d'aspiration, des moyens d'aspiration reliés audit volume d'aspiration pour créer une dépression à l'intérieur de ce volume, des moyens pour commander la mise en route et l'arrêt des moyens d'aspiration.

Dans un grand nombre d'activités industrielles, comme l'industrie nucléaire ou l'industrie chimique, le personnel peut se trouver au contact de substances minérales ou organiques agressives et/ou toxiques. La protection des membres de ces opérateurs, en particulier des mains, est donc nécessaire. A cet effet, on utilise, suivant les caractéristiques d'agressivité des corps ou des produits utilisés, des gants spéciaux d'épaisseur variable, allant des gants noirs de vinyle épais à des gants de latex blancs très minces, en passant par des gants de caoutchouc, d'épaisseur intermédiaire utilisés couramment dans le secteur nucléaire.

Le problème délicat, tant dans les laboratoires de recherche que dans l'industrie, n'est pas de mettre des gants neufs mais de retirer ces gants après une intervention. En effet, ces derniers peuvent être contaminés et il importe que l'opérateur puisse retirer ses gants sans que sa peau soit atteinte par cette contamination.

En plus des risques inhérents à la santé du travailleur, la contamination peut s'étendre par contact à l'environnement (rampes d'ecaliers, objets divers manipulés), ce qui nécessite des opérations de décontamination longues et coûteuses.

On connaît déjà (US-A-1 996 377) une machine permettant de mettre des gants élastiques, par exemple de caoutchouc. Elle comporte un boîtier, une enceinte formant boîte de dégantage située à l'intérieur du boîtier, et une paroi perforée située à l'intérieur de l'enceinte. Des moyens d'aspiration permettent de créer une dépression dans l'enceinte.

Toutefois, cette machine permet seulement de mettre les gants, mais elle ne permet pas de les enlever. Comme on l'a rappelé précédemment, en raison de la contamination extérieure, le problème le plus délicat n'est pas de mettre des gants neufs mais de retirer ces gants après une intervention.

En outre, pour que les gants puissent être enfilés sur la main, il est nécessaire que le rebord du gant soit au préalable étiré puis fixé sur le rebord de l'enceinte formant boîte de dégantage. La mise du gant, qui nécessite une opération manuelle n'est donc pas entièrement automatique.

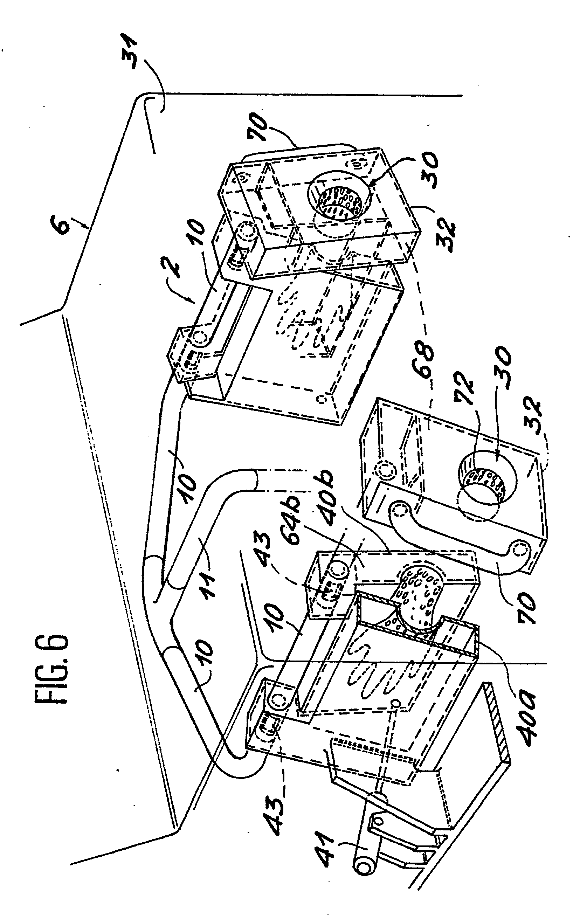

On connaît également (US-A-3 695 493) un dispositif pour enlever et mettre des gants sans contact avec l'extérieur. Contrairement à la machine citée précédemment, il permet effectivement de retirer des gants. Toutefois, il ne s'adapte pas à n'importe quel type de gants. En effet, il est nécessaire que ces derniers comportent un bourrelet (voir références 112,113 sur la figure 6 et la description correspondante, colonne 6, lignes 48-58). De plus, le volume intérieur de la boîte d'enlèvement ne s'ouvre pas de telle sorte que son accès est malaisé.

La présente invention a précisément pour objet une machine pour enlever et mettre des protections telles que des gants qui remédie aux inconvénients de la technique antérieure énumérés ci-dessus. Cette machine doit être entièrement automatique. Elle doit permettre de retirer un gant sans manipulation de ce dernier,en un temps très bref, de l'ordre de quelques secondes, sans aucun risque de contamination pour les mains de l'opérateur ou un autre de ses membres. Elle doit permettre également de confiner les gants ou autres protections retirées. En outre, elle doit s'adapter à tous les types de gants disponibles dans le commerce, que ceux-ci comportent ou non un bourrelet au niveau du poignet.

Par ailleurs, le volume de réception du membre comportant la protection (le bras ou la jambe) doit être étudié de manière à donner le maximum d'efficacité à l'aspiration.

A cet effet, l'invention est caractérisée en ce qu'elle comporte des moyens permettant de créer une dépression plus importante dans la zone d'entrée du volume de réception que dans le reste de la boîte d'enlèvement, de telle manière qu'une étanchéité est créée entre la protection à enlever et la zone d'entrée de la boîte d'enlèvement.

Selon une réalisation particulière, les perforations, situées dans la zone d'entrée présentent une section de passage relativement plus importante que dans le reste de la paroi perforée.

Selon une autre variante de réalisation, ces moyens sont constitués par un boîtier situé dans la zone d'entrée de ladite boîte d'aspiration, ce boîtier délimitant un volume d'aspiration distinct et séparé du volume d'aspiration de la boîte d'enlèvement.

Grâce à la présence des moyens permettant de créer une dépression plus importante dans la zone d'entrée du volume de réception que dans le reste de ce volume, le poignet du gant est collé par aspiration contre cette zone d'entrée avant le reste de la surface du gant, ce qui crée une étanchéité entre le poignet du gant et la zone d'entrée de la boîte d'enlèvement facilitant ensuite l'aspiration du reste de la surface du gant. En outre, cette zone d'aspiration préférentielle supprime la nécessité de recourir à un bourrelet ou d'étirer préalablement manuellement le rebord du gant pour le fixer sur la machine. La pose et la dépose du gant sont donc entièrement automatiques sans aucune manipulation. Elles peuvent donc être réalisées en un temps très bref, de l'ordre de quelques secondes. En outre, du fait que l'on évite toute manipulation manuelle, les risques de contamination pour les mains de l'opérateur ou un autre de ses membres sont supprimés.

En outre, le gant ayant été retiré, la machine doit permettre une évacuation aisée de ce gant. Elle doit permettre de plus un accès facile à l'intérieur de la boîte d'enlèvement afin de permettre sa décontamination.

Selon une autre caractéristique préférentielle, la paroi perforée est constituée de deux demi-coquilles montées pivotantes l'une par rapport à l'autre, la machine comportant en outre des moyens pour commander le pivotement de l'une au moins de ces demi-coquilles de manière à laisser un passage pour l'évacuation du gant.

Grâce à cette caractéristique, on peut accéder aisément à l'intérieur de la boîte de dégantage après l'enlèvement d'un gant dont la surface extérieure a été contaminée. Cette contamination s'étant transmise à la paroi intérieure de la surface de la boite de dégantage, il est nécessaire de procéder à la décontamination de cette dernière. Cette opération peut être effectuée facilement, avec la machine de l'invention, grâce au fait que l'accès à l'intérieur de la boîte de dégantage est facilité par son ouverture. On peut ainsi décontaminer aisément cette surface à l'aide d'un coton imbibé de produit décontaminant.

En outre, le fait que la boîte de dégantage s'ouvre en deux permet de prévoir un rétreint (col ou zone de plus faible section) au niveau du poignet. Il n'est en effet pas nécessaire que l'ouverture d'entrée de la boîte de dégantage présente une section suffisamment grande pour laisser passer la totalité de la main. Ainsi, la géométrie de la boîte à gants peut être conçue de manière à donner le maximum d'efficacité à l'aspiration au niveau du poignet. On comprend en effet aisément que plus la paroi est rapprochée du poignet, plus l'aspiration se fera de manière efficace. Cette caractéristique vient en complément de la caractéristique énoncée précédemment, selon laquelle l'invention comporte des moyens permettant de créer une dépression relativement plus importante dans la zone d'entrée du volume de réception de la boîte d'enlèvement.

Enfin, après l'ouverture de la boîte de dégantage, cette ouverture s'effectuant vers le bas, le gant est évacué aisément. Il peut être recueilli dans un sac, par exemple de matière plastique, et confiné par un procédé connu. Ainsi, on évite que la contamination ne se répande, ce qui constitue un avantage supplémentaire de l'invention.

D'autres modes particuliers de réalisation de l'invention apparaissent dans les revendications dépendantes 5 et 6.

D'autres avantages de la présente invention apparaîtront encore à la lecture de la description qui suit d'un exemple de réalisation donné à titre illustratif en référence aux figures annexées. Sur ces figures :

- la figure 1 représente une vue schématique de l'ensemble d'une machine à enlever les protections conforme à la présente invention ;
- les figures 2 à 5 illustrent le principe de fonctionnement d'une machine à enlever les protections conforme à la présente invention ;
- la figure 6 est une vue en perspective de la partie supérieure de la machine à enlever les protections représentée sur la figure 1 ;

- la figure 7a est une vue en coupe à travers la boîte de dégantage de la machine représentée sur les figures 1 et 6 en position fermée, et la figure 7b, en position ouverte ;
- la figure 8 est une vue en coupe d'une variante de réalisation d'une boîte de dégantage faisant partie d'une machine conforme à l'invention.

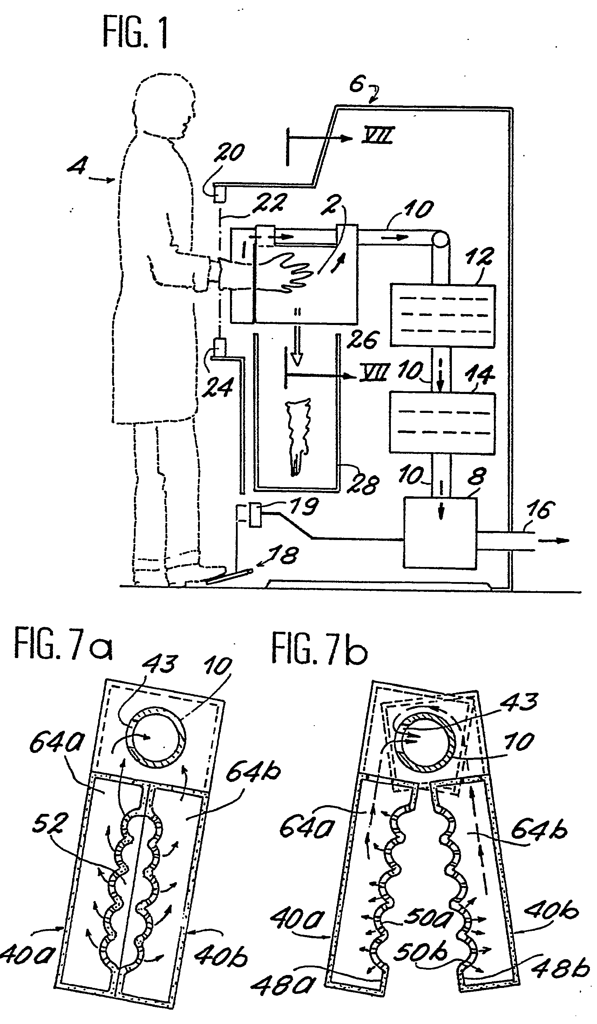

On a représenté sur la figure 1 une vue schématique d'une machine à enlever des protections conforme à la présente invention. Cette machine dont le carter 6 a été schématisé par un trait mixte, comporte une boîte d'enlèvement 2 dans laquelle la protection est enlevée. Cette protection peut être constituée par un gant ou une surbotte. Dans l'exemple de réalisation décrit, la machine est utilisée pour ôter des gants des mains d'un opérateur 4 debout devant la machine. La boîte 2 sera donc appelée boîte de dégantage dans la suite du texte. Sa structure sera expliquée plus en détail ultérieurement.

La boîte de dégantage 2 est reliée par une canalisation d'aspiration 10 à des moyens d'aspiration, schématisés par la référence 8. Dans l'exemple décrit, deux filtres, à savoir un préfiltre 12 et un filtre absolu 14 sont interposés entre la boîte d'enlèvement 2 et les moyens d'aspiration 8. Ces filtres sont particulièrement nécessaires dans l'industrie nucléaire pour piéger la contamination qui s'est déposée sur la surface extérieure du gant au cours des manipulations. De cette manière, l'air aspiré peut être rejeté sans inconvénient hors de la machine par la canalisation d'évacuation 16. Mais bien entendu, dans d'autres applications la machine peut fonctionner sans filtre à air.

La machine comporte des moyens pour commander la mise en route et l'arrêt des moyens d'aspiration 8. Selon une variante de réalisation, ces moyens sont constitués par une pédale 18 actionnée par l'opérateur 4 lui-même. Lorsque l'opérateur appuie sur la pédale, il ferme un interrupteur 19 qui alimente en courant électrique les moyens d'aspiration 8 qui sont alors mis en route. Inversement, lorsque l'opérateur relâche la pression exercée sur la pédale 18, le courant est coupé et l'aspiration cesse.

Selon une variante de réalisation, représentée sur la même figure par souci de simplification, les moyens pour commander la mise en route et l'arrêt des moyens d'aspiration 8 comprennent une source lumineuse 20 émettant un faisceau, schématisé par la flèche 22, dirigé vers une cellule photoélectrique 24. Lorsque l'opérateur introduit une (ou deux) main(s) à l'intérieur de la boîte de dégantage, le faisceau 22 est interrompu, ce qui provoque la mise en route des moyens d'aspiration 8 après un certain délai prédéterminé obtenu à l'aide d'un moyen de temporisation. Lorsque la ou les protections a été retirée de la main de l'opérateur de la manière qui sera expliquée ultérieurement, elle est évacuée, de préférence par gravité, comme schématisé par la flèche 26, dans un bac de réception 28.

De préférence, pour des applications nucléaires, un sac de vinyle est raccordé de manière étanche à la boîte de dégantage. Ce sac est scellé par des soudures thermiques réalisées à deux endroits différents. Une découpe est ensuite effectuée entre les deux soudures. Ainsi la contamination reste confinée dans le sac, sans pouvoir contaminer l'intérieur de la machine.

On a représenté sur les figures 2 à 5 quatre vues schématiques d'une boîte de dégantage 2 d'une machine à enlever les protections conforme à la présente invention à différents stades de fonctionnement. Ces quatre vues illustrent le principe de fonctionnement de la machine.

Schématiquement, la boîte de dégantage comporte une paroi perforée 50 qui divise son volume intérieur en deux zones, à savoir une zone 64, située à l'extérieur de la paroi perforée 50 et un volume de réception 52 dont la forme se rapproche le plus possible de celle d'une main 47. Le volume d'aspiration 64 est relié par la canalisation 10 aux moyens d'aspiration 8 (voir figure 1).

Sur la figure 3, la main 47 revêtue d'un gant 49 est introduite dans le volume de réception délimité par la paroi perforée 50. Dans cette position, le volume d'aspiration 64 est à la pression atmosphérique. En d'autres termes, aucune dépression n'y règne encore de telle sorte que le gant 49 adhère à la main sur toute sa surface.

La géométrie de la boîte de dégantage (secteur cylindrique) et la disposition des orifices calibrés ont été calculées pour assurer l'efficacité maximum.

Les moyens d'aspiration sont alors mis en route de telle sorte qu'une dépression apparaît dans le volume d'aspiration 64. Cette dépression se communique au volume de réception 52 par les trous de la paroi perforée. Une dépression plus importante apparaît dans la zone de poignet 51. Par suite, le gant 49 se décolle d'abord au niveau du poignet et vient se plaquer contre le pourtour cylindrique de l'entrée 30 du volume d'aspiration 52, ce qui a pour effet de créer une étanchéité entre l'entrée du volume 52 et la surface du gant 49 et d'une façon plus générale, l'air présent dans la boîte de dégantage est isolé de la pression atmosphérique de l'appareil.

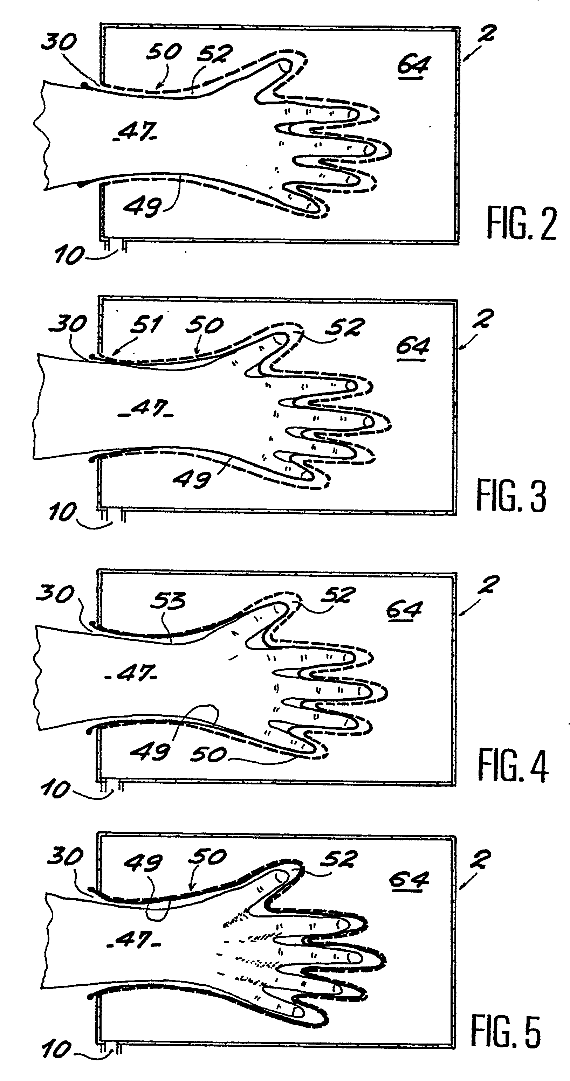

Sous l'effet de la différence de pression qui existe entre le volume d'aspiration 64 et l'intérieur du gant 49, par où la pression atmosphérique s'introduit par l'intérieur du gant, la paroi du gant s'étire et s'écarte progressivement de la main 47, d'abord au niveau de la paume et du dos (référence 53 sur la figure 4), puis enfin jusqu'au bout des doigts (voir figure 5).

La main de l'opérateur est ainsi totalement libérée et il peut la retirer sans difficulté.

On a représenté sur la figure 6 une vue en perspective de la partie supérieure de la machine à enlever des protections selon l'invention. Elle comporte deux boîtes de dégantage 2, comportant chacune une ouverture 30 ouvrant sur la face avant de la machine. Un conduit d'aspiration 10 est relié à chacune des boîtes 2. Ces deux conduits se rejoignent dans un tube collecteur 11 relié au préfiltre 12, puis au filtre 14. A la sortie du filtre 14, le collecteur 11 est relié à un groupe d'aspiration (non représenté).

Différentes réalisations de la boîte de dégantage sont possibles.

Selon le mode de réalisation représenté sur la figure 6, la boîte de dégantage 2 est constituée d'une part d'un boîtier 32 situé à l'avant de la boîte de dégantage par rapport à la face avant 31 de la machine, et d'autre part, de la boîte de dégantage proprement dite, cette boîte contenant en creux une empreinte en deux parties correspondant à la forme d'une main.

La fonction du boîtier 32 est de créer une aspiration plus importante au niveau du poignet de l'opérateur que dans le reste de la main afin de créer une étanchéité à l'air au niveau du poignet entre le gant et le pourtour de l'ouverture du boîtier 32, comme expliqué précédemment. Il comporte une chambre fermée 68 reliée à une canalisation d'aspiration 70 elle-même reliée aux moyens d'aspiration par l'intermédiaire de la canalisation d'aspiration 10 de la boîte de dégantage proprement dite. Cette réalisation améliore l'efficacité. Mais on pourrait bien évidemment prévoir une canalisation d'aspiration particulière pour le boîtier 32. La chambre d'aspiration 68 du boîtier 32 est séparée de celle de la boîte de dégantage proprement dite, ce qui facilite l'apparition d'une dépression plus importante au niveau du poignet que dans le reste de la boîte de dégantage. Pour augmenter encore cet effet, les perforations 72 pratiquées dans la zone d'entrée 30 présentent de préférence un diamètre plus grand que les perforations 52a et 52b de la boîte de dégantage.

On a décrit sur les figures 7a et 7b une vue en coupe de la boîte de dégantage de la machine à enlever les protections de la figure 6. La figure permet d'apprécier les deux demi-coquilles 40a, 40b articulées sur le tube d'aspiration 10 qui joue ainsi le rôle d'axe de pivotement. Comme on peut le voir sur la figure 6, la demi-coquille 40b est reliée à la tige d'un vérin 41 qui permet de commander l'ouverture et la fermeture de la boîte de dégantage de manière à laisser un passage pour l'évacuation du gant vers le bac de réception 28 (voir figure 1). La demi-coquille fixe 40a est montée inclinée par rapport au plan vertical d'un angle correspondant à l'inclinaison de la demi-coquille mobile 40b en position d'ouverture maximale, de manière à laisser une ouverture plus grande pour l'évacuation du gant. On remarque en outre que le tube d'aspiration comporte des lumières 43 qui mettent en communication les volumes d'aspiration 64a et 64b de chacune des demi-coquilles 40a et 40b. Ces lumières permettent la mise en dépression des demi-coquilles et sont obtenues lors de l'ouverture des boîtes de dégantage.

Une empreinte 50a, 50b est prévue dans chacune des parois planes 48a, 48b de manière à déterminer un volume interne 52 correspondant approximativement à la forme d'une main. On comprend que plus la forme de l'empreinte se rapprochera de celle d'une main, mieux l'aspiration sera répartie et le retrait des mains facilité. Toutefois, il n'est pas absolument nécessaire que cette forme reproduise de manière très fidèle le contour extérieur d'une main pour que l'aspiration puisse décoller la paroi du gant de la peau de l'opérateur. Une forme simplifiée, telle que celle qui est représentée sur les figures est suffisante. Chacune des parois des empreintes 50a, 50b comporte une série de perforations 52a, 52b destinées au passage de l'air.

Selon une variante de réalisation, afin de simplifier la fabrication, les empreintes peuvent être ambidextres, c'est-à-dire identiques pour les mains droite et gauche. Dans ce cas, il suffit que chaque empreinte comporte deux emplacements symétriques pour recevoir indifféremment le pouce d'une main droite ou d'une main gauche.

On utilise des moyens automatiques pour commander l'ouverture et la fermeture de la boîte de dégantage, ainsi que la mise en route et l'arrêt des moyens d'obturation. Ces moyens peuvent être constitués par la cellule photoélectrique 20 dont le faisceau est interrompu par les mains 47 de l'opérateur lorsqu'il les introduit dans les boîtes de dégantage 2. L'interruption du faisceau 22 provoque le démarrage du cycle d'aspiration avec un retard réglable de une à trois secondes. Le décollement du gant s'effectue, comme décrit précédemment, d'abord à la hauteur du poignet, puis au niveau de la paume et du dos de la main et enfin au niveau des doigts, jusqu'à libération complète de la main de l'opérateur. L'opérateur sentant que ses mains jouent librement dans les gants peut alors les retirer et la cellule photoélectrique provoque la coupure du circuit électrique des moyens d'aspiration. L'arrêt de ces moyens s'effectue progressivement. La dépression s'annule et le vérin temporisé 41, dont la tige est fixée à la demi-coquille mobile 40b provoque l'ouverture de chacune des boîtes de dégantage. Les gants tombent alors dans le bac de réception. Les boîtes de dégantage se referment sous l'action du vérin temporisé et la machine est prête pour un nouveau cycle d'aspiration. Dans ce mode de réalisation, les boîtes de dégantage sont en position fermée en période d'attente.

Selon une variante de réalisation (non représentée), l'évacuation des gants retirés s'effectue par une aspiration à l'extrémité postérieure de chaque boîte.

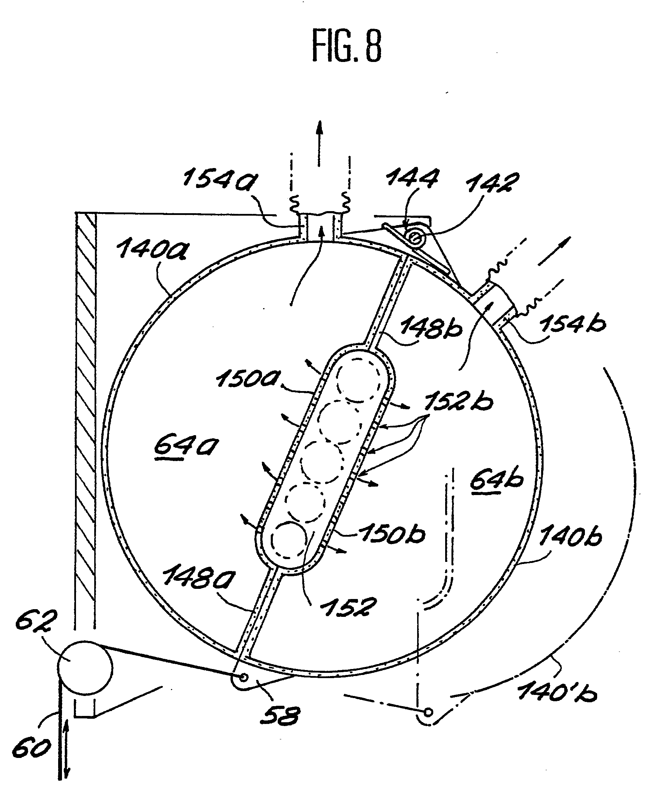

On a représenté sur la figure 8 une vue en coupe à travers une variante de réalisation d'une boîte de dégantage. Ce mode de réalisation est constitué de deux demi-coquilles 140a et 140b articulées par une charnière 142. Dans l'exemple de réalisation représenté, la demi-coquille 140a est fixe tandis que la demi-coquille 140b est mobile et peut venir occuper la position 140'b représentée en traits mixtes. Un ressort de rappel 144 sollicite en permanence la demi-coquille 140b vers sa position ouverte, c'est-à-dire vers la position 140'b représentée en traits mixtes.

Chaque demi-coquille 140a, 140b se compose d'une paroi demi-cylindrique et d'une paroi plane 148 disposée selon un plan diamétral du cylindre. Une empreinte 150a, 150b est prévue dans chacune des parois planes 148a, 148b de manière à déterminer un volume interne 152 correspondant approximativement à la forme d'une main. Chacune des empreintes 150a, 150b comporte une série de perforations 152a, 152b destinées au passage de l'air. En outre, une canalisation d'aspiration 154a, 154b est raccordée sur chacune des demi-coquilles 140a, 140b respectivement. Ces canalisations sont reliées à des moyens d'aspiration (non représentés). En outre, la demi-coquille 140b comporte une oreille 58 à laquelle est reliée une extrémité d'un câble 60 passant sur une poulie 62. L'autre extrémité du câble 60 est reliée à un moyen de commande, par exemple une pédale que l'opérateur peut actionner avec son pied. Lorsque la pédale n'est pas actionnée, la demi-coquille 140b se trouve dans la position représentée en traits mixtes. En d'autres termes, le volume de réception 152 dans lequel les mains sont introduites est ouvert, de manière à permettre le passage du gant vers un bac de réception (voir figure 1).

Le fonctionnement de ce mode de réalisation est le suivant :

L'opérateur enfonce ses mains gantées dans chacun des volumes de réception 152 des boîtes de dégantage, appuie la pédale de commande qui referme la boîte de dégantage (position représentée en trait plein sur la figure 8) et simultanément, ferme un interrupteur qui met en route les moyens d'aspiration avec un retard réglable de une à trois secondes. L'air contenu dans les volumes 64a et 64b est aspiré par les canalisations 154a et 154b de telle sorte qu'une dépression apparaît dans les volumes 64a et 64b. Cette dépression s'étend par les perforations au volume de réception et libère les mains de l'opérateur. L'opérateur, se rendant compte par contact tactile, que ses mains sont libérées, peut alors les retirer des boîtes de dégantage. Il relâche ensuite la pression exercée sur la pédale, l'aspiration s'arrête et la demi-coquille 140b est alors ramenée en position ouverte par le ressort de rappel 144, de telle sorte que les gants peuvent être évacués. L'arrêt de l'aspiration n'est pas instantané et la dépression s'annule progressivement. Puis les deux gants tombent dans le bac de réception 28. La boîte de dégantage reste en permanence en position ouverte jusqu'à une prochaine utilisation.

## Revendications

1. Machine à enlever des protections, comprenant au moins une boîte d'enlèvement (2), une paroi (50) comportant des perforations, située dans ladite boîte d'enlèvement et délimitant dans celle-ci d'une part un volume de réception (52) comportant une zone d'entrée (51) donnant sur l'extérieur de la boîte d'enlèvement (2) pour recevoir un membre humain (47) revêtu d'une protection (49), et d'autre part un volume d'aspiration (64), des moyens d'aspiration (8) reliés audit volume d'aspiration (64) pour créer une dépression à l'intérieur de ce volume, des moyens (18, 19, 20, 24) pour commander la mise en route et l'arrêt des moyens d'aspiration (8), caractérisé en ce qu'elle comporte des moyens (32, 72) permettant de créer une dépression plus importante dans ladite zone d'entrée (51) dudit volume de réception (52) que dans le reste de la boîte d'enlèvement (2), de telle manière qu'une étanchéité est créée entre la protection à enlever et la zone d'entrée (51) de la boîte d'enlèvement (2).

2. Machine selon la revendication 1, caractérisée en ce que les perforations situées dans la zone d'entrée (51) de ladite paroi perforée (50) présentent une section de passage relativement plus importante que les perforations situées dans le reste de ladite paroi perforée (50).

3. Machine selon la revendication 1 ou 2, caractérisée en ce que ces moyens sont constitués par un boîtier (32), situé dans la zone d'entrée (51) de ladite boite. d'aspiration, ce boîtier délimitant un volume d'aspiration (68) distinct et séparé de l'autre volume de la boîte d'enlèvement.

4. Machine selon l'une quelconque des revendications 1 à 3, caractérisée en ce que la paroi perforée (50) est constituée de deux demi-coquilles (40a, 40b, 140a, 140b) montées pivotantes l'une par rapport à l'autre, la machine comportant en outre des moyens (41, 60) pour commander le pivotement de l'une au moins de ces demi-coquilles, de manière à laisser un passage pour l'évacuation du gant (49).

5. Machine selon l'une quelconque des revendications 1 à 4, caractérisée en ce que le volume de réception (52) est adapté à recevoir une main revêtue d'un gant (49) et comporte une forme symétrique par rapport à un plan longitudinal de manière à pouvoir recevoir indifféremment une main droite ou une main gauche.

6. Machine selon l'une quelconque des revendications 1 à 5, caractérisée en ce qu'elle comporte des moyens d'aspiration situés à une extrémité dudit volume de réception (52) sensiblement opposée à la zone d'entrée (51), pour aspirer ladite protection.

## Patentansprüche

1. Gerät zum Entfernen von Schutzüberzügen mit: wenigstens einem Entfernungsgehäuse (2); einer Wand (50) mit Perforationen, die sich in dem Entfernungsgehäuse befindet und in diesem auf der einen Seite ein Aufnahmevolumen mit einer Eingangszone (51), die von dem Entfernungsgehäuse (2) nach außen geht, um ein menschliches Glied (47), das mit einem Schutzüberzug bedeckt ist, aufzunehmen, und auf der anderen Seite ein Ansaugvolumen (64) zu begrenzen; Ansaugvorrichtungen (8), die mit dem Ansaugvolumen verbunden sind, um einen Unterdruck im Innern dieses Volumens zu schaffen; Vorrichtungen (18, 19, 20, 24) zum Steuern des Startens und Anhaltens der Ansaugvorrichtungen (8); dadurch gekennzeichnet, daß es Vorrichtungen (32, 72) aufweist, die erlauben, in der Eingangszone (51) des Aufnahmevolumens (52) einen größeren Unterdruck als im Rest der Entfernungskammer (2) zu schaffen, um eine Dichtigkeit zwischen der zu entfernenden Schutzhülle und der Eingangszone (51) des Entfernungsgehäuses (2) zu bilden.

2. Gehäuse nach Anspruch 1, dadurch gekennzeichnet, daß die in der Eingangszone (51) der perforierten Wand (50) befindlichen Perforationen ei-nen relativ größeren Durchgangsquerschntt als die in dem Rest der perforierten Wand (50) befindlichen Perforationen aufweisen.

3. Gehäuse nach Anspruch 1 oder 2, dadurch gekennzeichnet, daß diese Vorrichtungen aus einer im Eingangsbereich (51) des Ansauggehäuses angeordneten Box (32) bestehen, wobei diese Box ein Ansaugvolumen (68) begrenzt, das von dem übrigen Volumen des Entfernungsgehäuses verschieden und getrennt ist.

4. Gehäuse nach einem der Ansprüche 1 bis 3, dadurch gekennzeichnet, daß die perforierte Wand (50) aus zwei zueinander schwenkbaren Halbschalen (40a, 40b, 140a, 140b) besteht, wobei das Gerät außerdem Vorrichtungen (41, 60) zum Steuern des Schwenkvorgangs wenigstens einer der Halbschalen umfaßt, um eine Öffnung für die Entleerung des Handschuhs (49) zu lassen.

5. Gehäuse nach einem der Ansprüche 1 bis 4, dadurch gekennzeichnet, daß das Aufnahmevolumen (52) geeignet ist, eine mit einem Handschuh (49) bekleidete Hand aufzunehmen, und eine symmetrische Form bezüglich einer longitudinalen Ebene besitzt, um ohne Unterschied eine rechte oder eine linke Hand aufnehmen zu können.

6. Gehäuse nach einem der Ansprüche 1 bis 5, da-durch gekennzeichnet, daß es Ansaugvorrichtun-gen umfaßt, die sich an einem Ende des Aufnahmevolumens (52) ziemlich genau gegenüber dem Eingangsbereich (51) befinden, um den Schutzüberzug anzusaugen.

## Claims

1. Machine for removing protections, comprising at least one removal box (2), a wall (50) which contains perforations, is situated in the said removal box and defines in the latter, on the one hand, a receiving space (52) having an entrance zone (51) open to the outside of the removal box (2) for receiving a human limb (47) covered by a protection (49) and, on the other hand, a suction space (64), suction means (8) connected to the said suction space (64) in order to create a reduced pressure inside this space, means (18, 19, 20, 24) for controlling the starting up and shutdown of the suction means (8), characterized in that it has means (32, 72) enabling a more substantial reduced pressure to be created in the said entrance zone (51) of the said receiving space (52) than in the remainder of the removal box (2) such that a leaktight fit is created between the protection to be removed and the entrance zone (51) of the removal box (2).

2. Machine according to Claim 1, characterized in that the perforations situated in the entrance zone (51) of the said perforated wall (50) have a relatively greater flow cross-section than the perforations situated in the remainder of the said perforated wall (50).

3. Machine according to Claim 1 or 2, characterized in that these means consist of a casing (32) situated in the entrance zone (51) of the said suction box, this casing defining a distinct suction space (68) separate from the other space of the removal box.

4. Machine according to any one of Claims 1 to 3, characterized in that the perforated wall (50) consists of two half-shells (40a, 40b, 140a 140b) mounted so as to pivot relative to each other, the machine furthermore having means (41, 60) for controlling the pivoting of at least one of these half-shells so as to allow a passage for the withdrawal of the glove (49).

5. Machine according to any one of Claims 1 to 4, characterized in that the receiving space (52) is adapted for receiving a hand covered with a glove (49) and has a symmetrical shape relative to a longitudinal plane so as to be able to receive equally well a right hand or a left hand.

6. Machine according to any one of Claims 1 to 5, characterized in that it has suction means situated at an end of the said receiving space (52) which is substantially opposite the entrance zone (51) in order to apply suction to the said protection.

FIG. 1

FIG. 7a    FIG. 7b

FIG. 2

FIG. 3

FIG. 4

FIG. 5

FIG. 6

# FIG. 8